# EUROPEAN PATENT SPECIFICATION

(11) **EP 4 033 990 B1**
(45) Date of publication and mention of the grant of the patent: **13.08.2025**
(21) Application number: 20785465.4
(22) Date of filing: 28.09.2020
(51) Int. Cl.: A61B 8/12, A61B 8/00

(54) **APPARATUS WITH INSERTION TUBE HAVING VARYING DUROMETER VALUE**
VORRICHTUNG MIT EINFÜHRUNGSROHR, DAS EINEN VARIIERENDEN DUROMETER-WERT AUFWEIST
APPAREIL AVEC TUBE D'INSERTION AYANT UNE VALEUR DE DUROMÈTRE VARIABLE

(30) Priority: 27.09.2019 US 201962906762 P
(43) Date of publication of application: 03.08.2022
(73) Proprietor: Koninklijke Philips N.V., 5656 AG Eindhoven (NL)
(72) Inventor: CUSCUNA, Dino, Francesco, 5656 AE Eindhoven (NL); CHAN, Edward, 5656 AE Eindhoven (NL); ORTIZ VAZQUEZ, Eduardo, 5656 AE Eindhoven (NL); LAGHI, Andrea, 5656 AE Eindhoven (NL); JINKS, Kathryn, Therese, 5656 AE Eindhoven (NL)
(74) Representative: Philips Intellectual Property & Standards
(86) International application number: PCT/EP2020/077022
(87) International publication number: WO 2021/058813

(56) References cited:
- US-A1- 2004 153 049
- US-A1- 2012 108 979
- US-A1- 2019 274 658

## Description

### FIELD

The invention relates to an apparatus with an insertion tube having a varying durometer value.

### BACKGROUND

US 2012/108979 A1 discloses an imaging catheter comprising a plurality of segments along the length of the catheter body. The catheter may be provided so that the stiffness of different ones of the plurality of segments may be different.

US 2019/274658 A1 discloses a lined variable braided differential durometer multi-lumen catheter shaft.

US 2004/153049 A1 discloses a catheter including a liner, a braid, and an outer jacket assembled to provide varying flexibility along a length of the catheter.

Transesophageal echocardiography (TEE) is one type of diagnostic medical procedure that uses ultrasound to create very clear images of the heart structure, valves and arteries. The TEE transducer is attached to a tube that is inserted through the mouth, down the throat and into the esophagus. Generally, the tube includes a bending neck on which the TEE transducer is disposed, and an insertion tube disposed between the bending neck and the clinician. During a procedure, the clinician guides the TEE transducer to a desired location in the esophagus by pushing on the insertion tube. To achieve clear images reliably, the TEE probe tip needs to remain stable and maintain good contact to the esophagus wall.

While TEE exams have relatively low complication rates, some common side effects are common in known TEE devices that cause discomfort for the patient during and after the procedure. Consequences of manipulating the TEE transducer to achieve desired images include discomfort from gagging, esophagus irritation, bleeding (rare) and sore throat post-procedure.

These discomforts are a result of comparatively large transducer heads and comparatively large diameter insertion tubes. While an overall smaller diameter TEE transducer is desirable as it would make intubation less traumatic and more comfortable for patients, image quality may be compromised due to poor contact of the TEE transducer tip with the esophagus using known components. Moreover, the known comparatively large diameter insertion tubes include all electrical elements (e.g., cables), as well as pull cables used to manipulate the position of the transducer head during the procedure.

While improvements have been made in TEE transducers that enable fewer electrical cables in the insertion tube (and thus a reduction of the outer diameter (OD) of the insertion tube), the transfer of forces by the clinician needed to properly guide and locate the TEE transducer can suffer due to an overall lower durometer value of known lower OD insertion tubes. As will be appreciated, proper location of the TEE transducer is important to obtaining the best available image quality.

What is needed, therefore, is a TEE transducer assembly that reduces irritation and patient discomfort, while providing high quality images.

### SUMMARY

The invention is defined by the independent claims. The dependent claims define advantageous embodiments.

### BRIEF DESCRIPTION OF THE DRAWINGS

The representative embodiments are best understood from the following detailed description when read with the accompanying drawing figures. It is emphasized that the various features are not necessarily drawn to scale. In fact, the dimensions may be arbitrarily increased or decreased for clarity of discussion. Wherever applicable and practical, like reference numerals refer to like elements.
Fig. 1A is a partial cross-sectional view of an apparatus inserted down an esophagus of a patient in accordance with a representative embodiment.
Fig. 1B is a perspective view of the apparatus of Fig. 1A.
Fig. 1C is a perspective view of an apparatus in accordance with a representative embodiment.
Fig. 2A is a perspective view of an insertion tube connected to a bending neck for use in an apparatus in accordance with a representative embodiment.
Fig. 2B is a cross-sectional view of the insertion tube of Fig. 2A along the line 2B-2B.
Fig. 2C is a cross-sectional view of the insertion tube of Fig. 2A along the line 2C-2C.
Fig. 2D is a perspective view of a monocoil used for an inner jacket of an insertion tube in accordance with a representative embodiment.
Fig. 3A is a perspective view of insertion tube in accordance with a representative embodiment.
Fig. 3B is a partial cross-sectional view of an apparatus inserted down an esophagus of a patient in accordance with a representative embodiment.
Fig. 4A is a side view of a portion of a braid having a gradual change in braid angle along its length in accordance with a representative embodiment.
Fig. 4B shows a section of the braid and the braid angle at the section of the braid shown in Fig. 4A.
Fig. 4C shows another section of the braid and the braid angle at the other section of the braid shown in Fig. 4A.
Fig. 4D is a side view of a portion of a braid having a continuous change in braid angle along its length.

### DETAILED DESCRIPTION

In the following detailed description, for purposes of explanation and not limitation, representative embodiments disclosing specific details are set forth in order to provide a thorough understanding of an embodiment according to the present teachings. Descriptions of known systems, devices, materials, methods of operation and methods of manufacture may be omitted so as to avoid obscuring the description of the representative embodiments but may be used in embodiments. It is to be understood that the terminology used herein is for purposes of describing particular embodiments only and is not intended to be limiting. The defined terms are in addition to the technical and scientific meanings of the defined terms as commonly understood and accepted in the technical field of the present teachings.

It will be understood that, although the terms first, second, third etc. may be used herein to describe various elements or components, these elements or components should not be limited by these terms. These terms are only used to distinguish one element or component from another element or component. Thus, a first element or component discussed below could be termed a second element or component without departing from the teachings of the inventive concept.

The terminology used herein is for purposes of describing particular embodiments only and is not intended to be limiting. As used in the specification and appended claims, the singular forms of terms 'a', 'an' and 'the' are intended to include both singular and plural forms, unless the context clearly dictates otherwise. Additionally, the terms "comprises", and/or "comprising," and/or similar terms when used in this specification, specify the presence of stated features, elements, and/or components, but do not preclude the presence or addition of one or more other features, elements, components, and/or groups thereof. As used herein, the term "and/or" may be used and includes any and all combinations of one or more of the associated listed items.

Unless otherwise noted, when an element or component is said to be "connected to", another element or component, it will be understood that the element or component can be directly connected to the other element or component, or intervening elements or components may be present. That is, these and similar terms encompass cases where one or more intermediate elements or components may be employed to connect two elements or components.

Various apparatus are described herein for use in ultrasound imaging. In accordance with various representative embodiments, the apparatuses may be a TEE assembly, or an ultrasound intra-cardia echo (ICE) imaging assembly, or an intravascular ultrasound (IVUS) assembly. It is emphasized that the present teachings are not limited to the noted assemblies, and may be applied to other ultrasound devices within the purview of one of ordinary skill in the art, having had the benefit of the present disclosure.

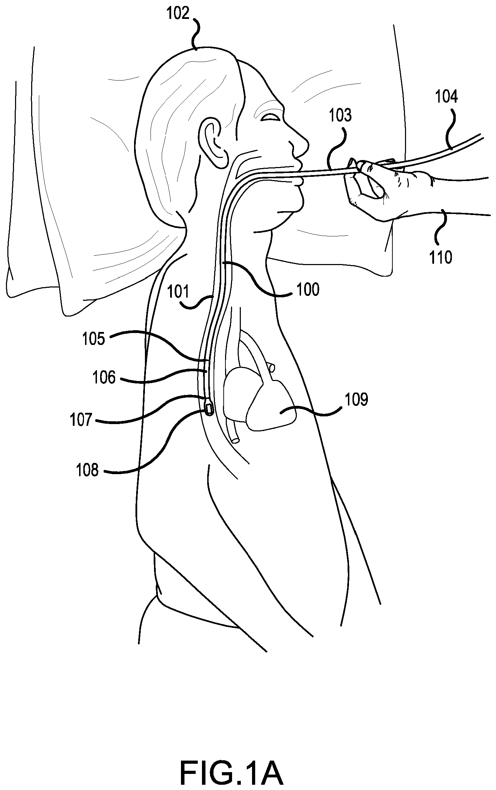

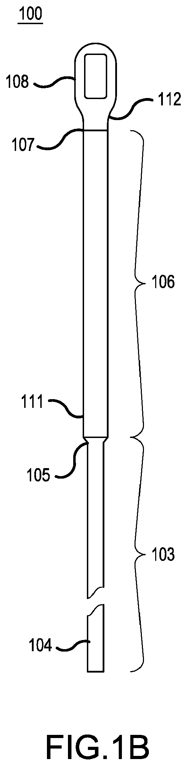

Fig. 1A is a partial cross-sectional view of an apparatus 100 inserted down an esophagus 101 of a patient 102 in accordance with a representative embodiment. The apparatus 100 comprises an insertion tube 103 at a proximal end 104. A distal end 105 of the insertion tube 103 is connected to a proximal end (see Fig. 1B) of a bending neck 106. A distal end 107 of the bending neck 106 is connected to a proximal end (see Fig. 1B) of a transducer assembly 108. Accordingly, the transducer assembly 108, the bending neck 106, and the insertion tube 103 are disposed in tandem.

The transducer assembly 108 is positioned in the esophagus 101 to foster high-quality echocardiography images of a heart 109 of the patient. As described more fully below, the proximal end 104 of the insertion tube 103 is manipulated by a clinician's hands 110 to guide the transducer assembly 108 in position for image gathering.

As will become clearer as the present teachings continue, the components of the apparatus 100 have dimensions that are smaller than the esophagus 101, but does not compromise image quality. To this end, the insertion tube 103, the bending neck 106, and the transducer assembly 108 each have a diameter or cross-sectional dimension that is less than the diameter or the cross-sectional dimension of the esophagus 101. However, as explained more fully below, the manipulation of the insertion tube 103, the bending neck 106 and the transducer assembly 108 by a clinician's hands 110 are not compromised by the reduced dimensions of the components of the apparatus 100 compared to known TEE assemblies, which have comparatively large components.

The transducer assembly 108 may be as described in WO 2018/06254A1 entitled "Intraluminal Imaging Devices with a Reduced Number of Signal Channels". Notably, the transducer assembly 108 as described in that application reduces the number of electrical cables needed for signal and power transmission to/from the transducer assembly 108, while maintaining the needed number of pull cables. This reduction in electrical cables, when compared to known TEE assemblies, fosters a reduced diameter/cross-sectional area needed to provide all needed electrical and mechanical connections from the ultrasound equipment (not shown) through the insertion tube 103 and the bending neck 106 to the transducer assembly 108. Moreover, compared to certain known transducer assemblies, the transducer assembly 108 has a smaller diameter/cross-section.

The reduction of dimensions of the insertion tube 103, the bending neck, and the transducer assembly 108 of the present teachings, compared to known components of TEE assemblies, reduces contact of these components of the apparatus 100 with the esophagus, and beneficially improves patient comfort. As described more fully below, however, the improvements of the apparatus 100 in comfort do not compromise image quality since dexterity of placement of the transducer assembly 108 in a location needed for proper imaging of the heart 109 and its surrounding structures are not compromised.

Fig. 1B is a perspective view of apparatus 100 in accordance with a representative embodiment. Many aspects of the apparatus 100 are common to those described in connection with Fig. 1A, and may not be repeated.

The apparatus 100 comprises insertion tube 103 having proximal end 104. The distal end 105 of the insertion tube 103 is connected to a proximal end 111 of a bending neck 106. The distal end 107 of the bending neck 106 is connected to a proximal end 112 of the transducer assembly 108. Accordingly, the transducer assembly 108, the bending neck 106, and the insertion tube 103 are disposed in tandem.

In accordance with a representative embodiment, the stiffness of the proximal end 104 of insertion tube 103 is greater than the stiffness of the distal end 105 of the insertion tube 103. This comparatively high durometer value of the proximal end 104 of the insertion tube 103 provides a rigidity that is greater than the rigidity of the distal end 105 of the insertion tube 103. As will be appreciated by one of ordinary skill in the art, it is beneficial to have a comparatively high durometer value for the proximal end 104 of the insertion tube 103 in order to properly transfer torque from the user to the insertion tube 103 to enable manipulation and articulation of the transducer assembly 108 as far as its distal end.

In accordance with a representative embodiment along its length, the insertion tube 103 has a durometer value in the range of approximately 50 Shore D to approximately 80 Shore D, with the higher value at the proximal end 104 and the lower value at the distal end 107.

Accordingly, the stiffness and thus the relative rigidity of the apparatus 100 decreases along its length from the proximal end 104 of insertion tube 103 to the distal end of the insertion tube. The change in stiffness along the length of the insertion tube 103 can be comparatively continuous, continual, or abrupt. As described more fully below, the insertion tube 103 can be fabricated so the stiffness decreases continuously or continually from its proximal end 104 to its distal end 105.

It is noted that in combinations of the above-described components of the apparatus 100, the stiffness of the bending neck 106 and the transducer assembly 108 are generally fixed, and driven by the insertion tube 103. As described more fully below for example, the insertion tube 103 may be fabricated so its stiffness continuously, continually, or abruptly decreases between its proximal end 104 and its distal end 105. This insertion tube 103 may be connected in tandem to the bending portion that has a constant stiffness between its proximal end 111 and its distal end 107. Taking this example further, the change in stiffness from the proximal end 104 to the distal end 105 of the insertion tube 103 can be made so there is no change in stiffness from the distal end 105 of the insertion tube 103 to the proximal end 111 of the bending neck 106 (i.e., the durometer value at the distal end 105 of the insertion tube 103 is substantially identical to the durometer value at the proximal end 111 of the bending neck 106).

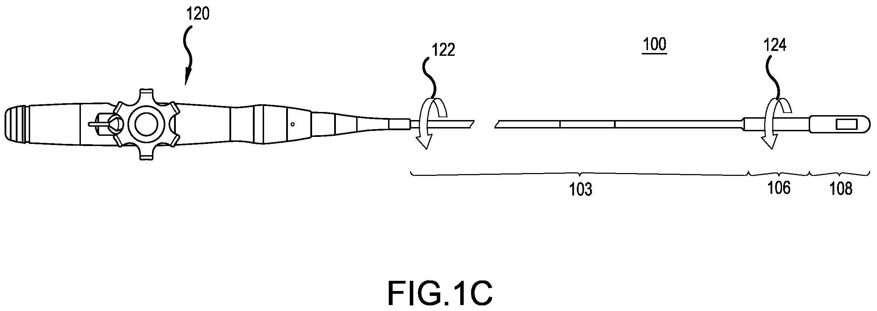

Regardless of whether the decrease in the durometer value between the proximal end 104 to the distal end 105 of the insertion tube 103 is continuous, or continual, or abrupt, the durometer value along the length of the insertion tube 103 is selected to transfer sufficient torque along the insertion tube 103, which has a reduced diameter/cross-section compared to known insertion tubes. This transfer of force is illustrated in Fig. 1C, which depicts apparatus 100 connected to a handle end 120. To this end, rotation 122 by of the handle end 120 by the user rotates the proximal end 104 of the insertion tube 103 and transfers torque at the bending neck 106 and transducer assembly 108, resulting in a rotation 124 of the transducer assembly 108.

The higher stiffness of the proximal end 104 of the insertion tube 103 compared to those of distal end 105 enables sufficient transfer of torque from the clinician at the proximal end 104 of the insertion tube 103 to the bending neck 106 without sacrificing flexibility in the bending neck 106 and transducer assembly 108 to move within the esophagus. As such, by the present teachings, a more patient-friendly apparatus 100, having a smaller cross-section is provided, while allowing allows proper articulation of the transducer assembly 108 in order to provide better quality images. By contrast to the present teachings, reducing the diameter/cross-section of known insertion tubes to those of the present teachings, while potentially beneficial to patient comfort, result in an unacceptably low durometer value, and ultimately unacceptable dexterity of the transducer assembly to enable images of acceptable quality.

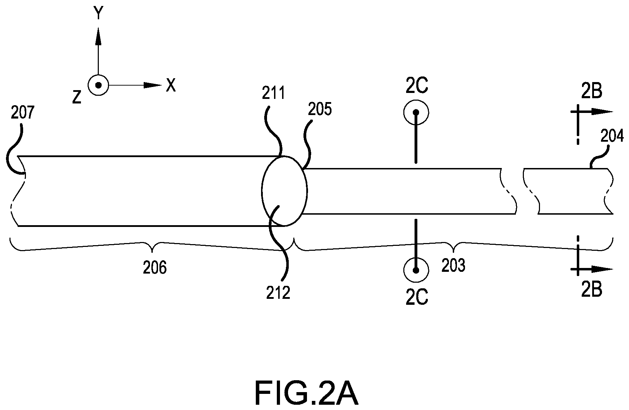

Fig. 2A is a perspective view of a bending neck 206 connected to an insertion tube 203 in accordance with a representative embodiment. Certain aspects of the bending neck 206 connected to an insertion tube 203 are common to those described in connection with representative embodiments described in connection with Figs. 1A-1C, and may not be repeated.

The insertion tube 203 has a proximal end 204 and a distal end 205. The distal end 205 of the insertion tube 203 is connected to a proximal end 211 of a bending neck 206. In a representative embodiment, a suitable adhesive 212, such as an epoxy that may be used in invasive medical procedures, is used to adhere the distal end 205 of the insertion tube 203 to the proximal end 211 of the bending neck 206. As will be appreciated, a distal end 207 of the bending neck 206 is connected to a proximal end (not shown in Fig. 2A) of a transducer assembly (not shown in Fig, 2A). Accordingly, the transducer assembly, the bending neck 206, and the insertion tube 203 are disposed in tandem.

In a representative embodiment, the bending neck 206 and insertion tube 203 are cylindrical in shape, and have a diameter measured along the z-axis of the coordinate system of Fig. 2A. As noted above, and as described more fully herein, the diameter of the insertion tube 203 is smaller than the diameter of the bending neck 206, and may be beneficially smaller than the diameter of known insertion tubes, while having a durometer value that is at least as great as known insertion tubes. More generally, the cross-sectional area (measured in the y-z plane of the coordinate system of Fig. 2A) of the insertion tube 203 is less than the cross-sectional area of the bending neck 206, and may be beneficially smaller than the diameter of known insertion tubes, while having a durometer value that is at least as great as known insertion tubes.

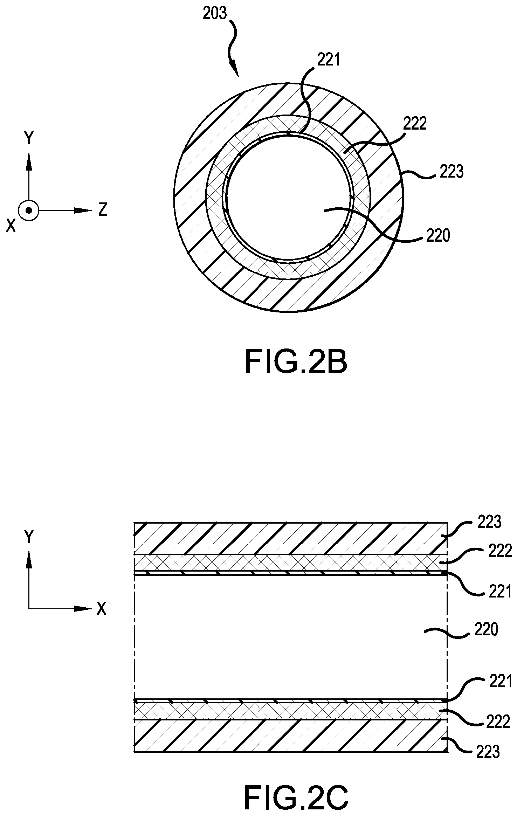

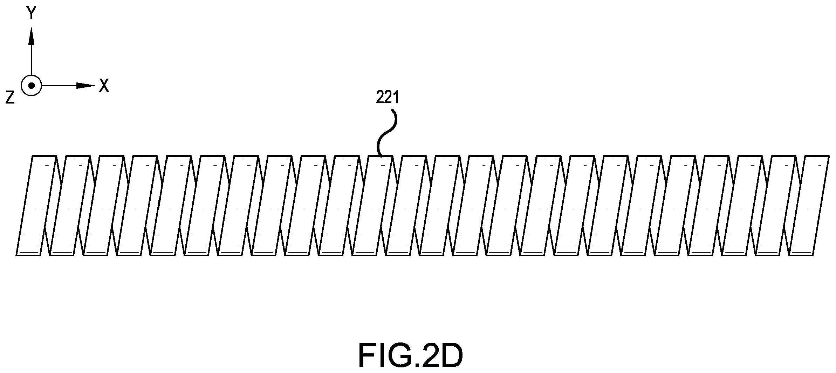

Fig. 2B is a cross-sectional view of the insertion tube 203 of Fig. 2A along the line 2B-2B. Fig. 2C is a cross-sectional view of the insertion tube 203 of Fig. 2A along the line 2C-2C. Fig. 2D is a perspective view of an inner jacket 221 in accordance with a representative embodiment. Certain aspects of the insertion tube 203 are common to those described in connection with representative embodiments described in connection with Figs. 1A-2A, and may not be repeated.

As shown in Figs. 2B and 2C, the insertion tube 203 has an interior channel 220, which is configured to hold an electrical wire (not shown) used to transmit signals to/from the transducer assembly (not shown in Fig. 2B), as well as pull cables (not shown) used for manipulation of the transducer assembly during a medical procedure. Illustratively, the interior channel 220 has a diameter of approximately 4 mm to approximately 5 mm.

In a representative embodiment, an inner jacket 221 is disposed circumferentially around the interior channel 220. As described in greater detail in connection with Fig. 2D, and more fully below, the inner jacket 221 may be used to provide a desired stiffness along a length (x-direction in the coordinate system of Fig. 2B) of the insertion tube 203.

Circumferentially surrounding the inner jacket 221 is a braid 222. As described more fully below, the braid 222 may also be used to provide to provide a desired stiffness along a length (x-direction in the coordinate system of Figs. 2B-2D) of the insertion tube 203.

Circumferentially surrounding braid 222 is an outer jacket 223. As described more fully below, the outer jacket 223 may also be used to provide to provide a desired durometer value along a length (x-direction in the coordinate system of Figs. 2B-2D) of the insertion tube 203.

In accordance with representative embodiments of the present teachings, each of, or one or more of, the inner jacket 221, the braid 222, and the outer jacket 223 are used selectively to provide a desired durometer value along the length of the insertion tube. As explained more fully below, not only can each of the inner jacket 221, braid 222, and outer jacket 223 be selected for placement along a length (x-direction of Figs. 2B-2D) of the insertion tube 203 to provide a desired durometer value, but also their interaction along the length of the insertion tube 203 may be used to tailor the magnitude of the stiffness of the insertion tube 203 along its length. As described more fully below, through the use of one or more of the inner jacket 221, braid 222, and outer jacket 223, the desired stiffness magnitude, change in magnitude in stiffness, and type of change in magnitude (i.e., continuous, continual, discrete) in the stiffness along the length of the insertion tube 203 can be realized.

Returning to Fig. 2A, therefore, in accordance with various representative embodiments, the stiffness of the insertion tube 203 at its distal end 205 can be less than at its proximal end 204, with the inner jacket 221, braid 222, and outer jacket 223 used to tailor the desired durometer magnitude, change in magnitude, and type of change in magnitude (i.e., continuous, continual, discrete) in the stiffness of the insertion tube 203 between its proximal end 204 and distal end 205. Moreover, and as described more fully below, the inner jacket 221, braid 222, and outer jacket 223 may be used in combination, or selectively omitted, or both, to provide the desired magnitude, change, and type of change in the stiffness of the insertion tube 203 between its proximal end 204 and distal end 205.

In accordance with a representative embodiment, and as shown in Fig. 2D, the inner jacket 221 is a monocoil with a helical shape. The inner jacket 221 is used to provide a stiffer durometer value at the proximal end 204 than at the distal end 205 of the insertion tube 203, thereby improving, compared to known insertion tubes, the ability to transfer torque from the proximal end 204 to the distal end 205 of the insertion tube 203 without losing flexibility needed to move the transducer assembly (e.g., transducer assembly 108 of Figs. 1A-1B) within the body. Notably, and in accordance with a representative embodiment, the stiffness of the durometer provided by the inner jacket 221 can be increased by stacking more than one (e.g., two) inner jacket 221 upon each other at locations along the length (x-direction in the coordinate system of Figs. 2B-2D) where an comparatively stiff durometer. So, to increase the durometer value along the proximal end 204 of the insertion tube 203, two (or more) inner jackets 221 may be stacked upon one another along the desired length of the proximal end 204 of the insertion tube. By contrast, the relative stiffness of the insertion tube 203 at the distal end 205 can be desirably reduced by foregoing the inner jacket 221 in one or more portions of the insertion tube 203.

Illustratively, the inner jacket 221 comprising the monocoil is made of a suitable metal or metal alloy. Alternatively, the monocoil may be made of a synthetic material such as Kevlar^{®}. The gauge or thickness of the material used for the monocoil is selected to provide a desired rigidity and durometer value along the length (x-direction in the co ordinate system of Fig. 2B, 2C) of the monocoil and inner jacket. Similarly, the pitch of the helix (see Fig. 2D) of the monocoil can be altered to provide a desired rigidity and durometer value along the length of the monocoil, and thus the inner jacket. As such, the stiffness of the inner jacket 221 can be varied along the length (e.g., from the proximal end 204 to the distal end 205) of the insertion tube 203 by selection of the gauge or thickness, or pitch of the monocoil, or both, by selectively stacking and/or foregoing the monocoil or combinations thereof, at selected portions of along the length of the monocoil that comprises the inner jacket 221. By way of illustration and not limitation, the monocoil configuration may comprise flat wire having a width of approximately 1 mm to approximately 2 mm and a thickness of approximately 0.1 mm to approximately 0.2 mm. The wraps of the monocoil can range from touch (i.e., no gap) to spaced wraps having a gap as great as 5 mm. Alternately, the shape of the monocoil can be round or elliptical.

As alluded to above, in accordance with a representative embodiment, the braid 222 may also be used to provide a desired stiffness along the length (x-direction in coordinate system of Figs. 2B and 2C). Like the inner jacket 221, the braid 222 is used to provide a stiffer durometer at the proximal end 204 than at the distal end 205 of the insertion tube 203, thereby improving, compared to known insertion tubes, the ability to transfer torque from the proximal end 204 to the distal end 205 of the insertion without losing flexibility needed to move the transducer assembly (e.g., transducer assembly 108 of Figs. 1A-1B) within the body.

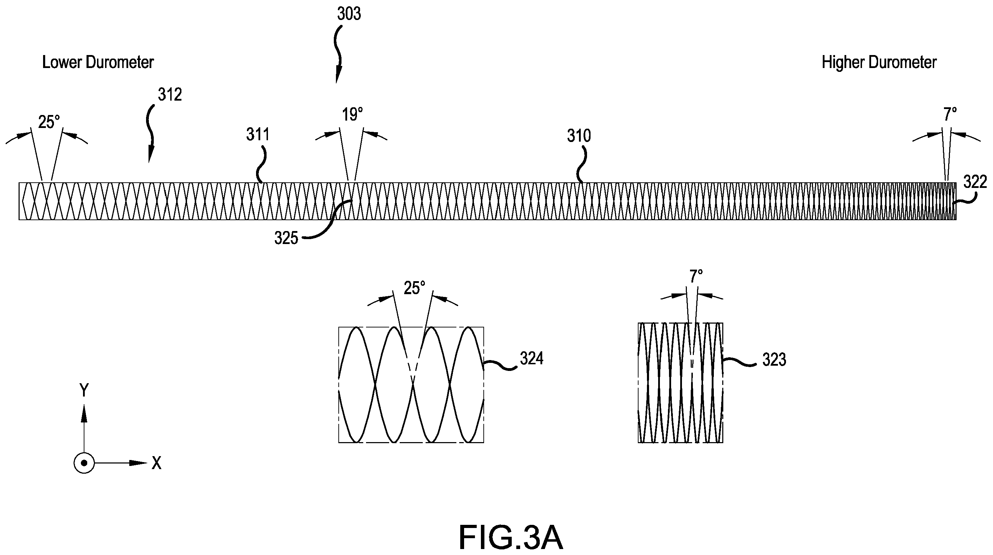

As described more fully below in connection with Figs. 3A-3B, the stiffness of the braid 222 can be selected along a length (x-direction of Figs. 2B-2C) of the insertion tube 203. Illustratively, the braid angle and the tensile modulus of the individual wires that make up the braid 222 can be selected to provide a desired stiffness at a location along the braid 222, and to alter the stiffness of the braid continually, continuously, or abruptly along the length of the insertion tube 203.

Illustratively, the braid 222 is made of a suitable metal or metal alloy. Alternatively, the monocoil may be made of a synthetic material such as Kevlar^{®}. As described more fully below in connection with Figs. 3A-3B, the braid angle, or the tensile modulus of the material used for the wires of the braid 222, or both, are selected to provide a desired rigidity and durometer value along the length (x-direction in the coordinate system of Fig. 2B, 2C) of the monocoil and inner jacket 221.

In accordance with a representative embodiment, the outer jacket 223 may also be used to provide a desired durometer value along the length (x-direction in coordinate system of Figs. 2B and 2C). Notably, the in certain embodiments, the braid 222 is adhered to an outer jacket 223 with adhesive. In this way, the braid 222 can be captured between the inner jacket 221 and the outer jacket 223 as a separate layer. Alternatively, the braid 222 is thermally bonded to the outer jacket 223. By way of example, the outer jacket 223 can be laminated to the braid 222 via co-extrusion or other known methods.

Like the inner jacket 221 and the braid 222, the outer jacket 223 is used to provide a stiffer durometer value at the proximal end 204 than at the distal end 205 of the insertion tube 203, thereby improving, compared to known insertion tubes, the ability to transfer torque from the proximal end 204 to the distal end 205 of the insertion tube 203 without losing flexibility needed to move the transducer assembly (e.g., transducer assembly 108 of Figs. 1A-1B) within the body.

Like the inner jacket 221 and the braid 222, the stiffness of the outer jacket 223 can be selected along a length (x-direction of Figs. 2B-2C) of the insertion tube 203. Illustratively, as noted above, the durometer value of the outer jacket 223 can be made to vary continually, continuously, or abruptly along the length of the insertion tube 203. Construction of the desired stiffness profile along the length of the insertion tube 203 can be achieved by attaching various tubing by adhesive or thermally bonding or alternately via continuous variable extrusion methods. Just by way of illustration, the outer jacket 223 may comprise tubing commercially available from Putnam Plastics, Dayvillle, CT (USA). The stiffness of the outer jacket along the length of the insertion tube 203 using Total Intermittent Extrusion (TIE^{™}) by Putnam Plastics. This proprietary process is capable of producing extrusions with variable durometer values along their length. (http://www.putnamplastics.com/extrusions/intermittent-extrusion-tie).) As such, the magnitude of the stiffness of the insertion tube 203 along the proximal end 204 can be made greater than at the distal end 205 of the insertion tube 203 by the selection of the extrusion to provide the greater and lesser durometer values at the proximal end 204 and the distal end 205, respectively. Moreover, the extrusion process can be used to provide an outer jacket 223 having a durometer value that varies continually, continuously, or abruptly along the length of the insertion tube 203.

As noted above, each of the inner jacket 221, the braid 222, and the outer jacket 223 can be used selectively to provide the desired stiffness profile along the length (x-direction of Figs. 2B-2D) of the insertion tube 203. To this end, the braid 222, and the outer jacket 223 can be used or foregone along portions of the length of the insertion tube 203 to provide desired durometer magnitudes along the length of the insertion tube 203. Moreover, the selected stiffness of the outer jacket 223 can be selected to provide a desired durometer value at locations along the length of the insertion tube 203. As such, by the selective incorporation of the outer jacket 233 with the braid 222, or the inner jacket 221, the stiffness along the length of the insertion tube 203 can be varied as desired. As such, the insertion tube 203, having a variable durometer value along its length, can be realized by using the outer jacket 233 alone; or by incorporating the braid 222, or the inner jacket 221, or both, with the outer jacket 223.

The flexibility of the insertion tube 203 can also be tailored with combinations by selection of the durometer value of the plastic/polymer materials, and the modulus of the reinforcement metal materials such as the monocoil. For example, the durometer value of the inner jacket 221, or the outer jacket 223, or both, can be substantially constant along the length (along the x-axis in Figs. 2B, 2C) of the insertion tube 203, and the stiffness of the insertion tube 203 can be altered by varying the characteristics of the braid 222. This variation of the durometer can be made by selection of the monocoil material, stress modulus, shape, or configuration (e.g., round monocoil, flat monocoil, and spacing of coils of the monocoils) of the braid 222. Alternatively, the properties and configurations of the inner jacket 221, the outer jacket 223 and the monocoil can be varied to realize the desired durometer value profile along the length (along the x-axis in Figs. 2B, 2C) of the insertion tube 203.

Fig. 3A is a perspective view of insertion tube 303 in accordance with a representative embodiment. Notably, for ease of description, the outer jacket (e.g., outer jacket 223 of Fig. 2C) and the inner jacket (e.g., inner jacket 221 of Fig. 2C) are not shown in Fig. 3A in order to facilitate the description of the braid 322 along the length of the insertion tube 303. Certain aspects of the insertion tube 303 are common to those described in connection with representative embodiments described in connection with Figs. 1A-2C, and may not be repeated.

The insertion tube 303 has a first section 310, a middle section 311, and a second section 312. The first section 310 has a comparatively high durometer value; the middle section 311 has a lower durometer value than the first section 310; and the second section 312 has a durometer value that is lower than the middle section 311 or the first section 310.

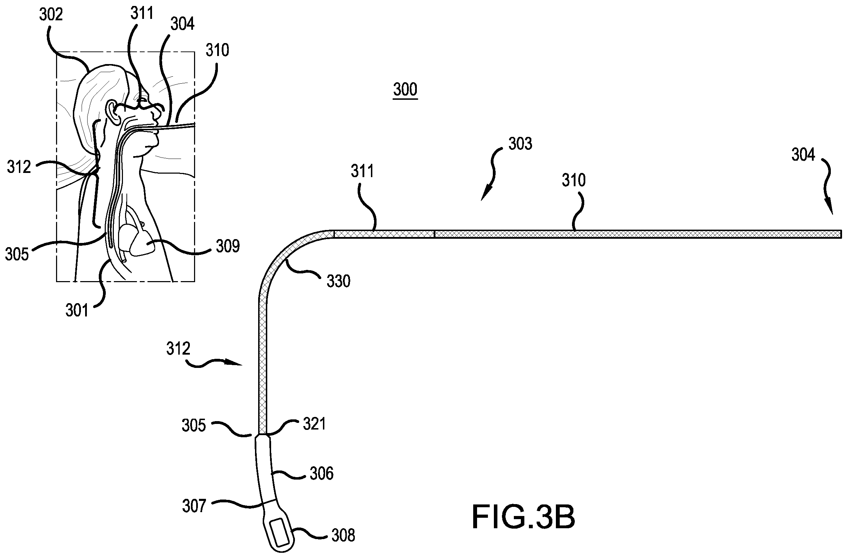

As noted above, the braid angle is one determinant of the durometer value of the braid 322. As shown in the inset of Fig. 3B, a first braid 323 having a braid angle of 25° and a second braid 324 has a braid angle of 7°. As is known, the rigidity (and thus the durometer value) of a braid is inversely dependent upon the braid angle. As such, the second braid 324 has a greater durometer value (and therefore is stiffer) than the first braid 323. Similarly, a third braid 325 having, an illustrative braid angle of 19° is between the braid angles of the first braid 323 and the second braid 324, is disposed in the middle section 311. As such, the middle section 311has a durometer value that is greater than that of the first braid 323 and less than that of the second braid 324.

In the presently described embodiment, the braid angle of the first section 310 is less than the braid angle of the middle section 311, and even less than the braid angle of the second section 312. This provides the first section 310 with a comparatively high durometer value; the middle section 311 with a lower durometer value than the first section 310; and the second section 312 with a durometer value that is lower than the middle section 311 or the first section 310. It is emphasized that the braid angle is only one of the factors that impacts the durometer value of a braid 322. As noted above, the tensile strength of the individual wires or fibers of the braid 322 also impact the magnitude of the durometer value of the braid 322. By the present teachings, the durometer value of the braid 322 is set by the selection of the braid angle, or by selection of the tensile strength of the braid 322, or both.

Notably, and as shown by the abrupt increase of the braid angle as shown, the change in durometer value between the first section 310 and the middle section 311 is comparatively abrupt. Similarly, the change in stiffness between the middle section 311 and the second section 312 is also abrupt, as evidenced by the abrupt increase in the braid angle of the second section 312 compared to the middle section 311. This is merely illustrative. In other representative embodiments described below, a continuous change in the braid angle of the braid 322 can be used to provide a change in the stiffness from the first section 310 to the middle section 311 and the second section 312.

Additionally, the change in the braid angle can be continual along the length (x-direction of Fig. 3A) of the insertion tube 303. To this end, and as described more fully below, rather than an abrupt change in braid angle between the first section 310 and the middle section 311, or between the middle section 311 and the second section 312, or both, or a continuous change in the braid angle along the length (x-direction of Fig. 3A) between the first section 310 and the second section 312, the braid angle can change gradually in the regions near the first section 310 and the middle section 311, or near the middle section 311 and the second section 312, or both.

Fig. 3B is a partial cross-sectional view of an apparatus 300 inserted down an esophagus 301 of a patient 302 in accordance with a representative embodiment. Certain aspects of the insertion tube 303 are common to those described in connection with representative embodiments described in connection with Figs. 1A-3A, and may not be repeated.

The apparatus 300 comprises an insertion tube 303 at a proximal end 304. A distal end 305 of the insertion tube 303 is connected to a proximal end 321 of a bending neck 306. A distal end 307 of the bending neck 306 is connected to a proximal end of a transducer assembly 308. Accordingly, the transducer assembly 308, the bending neck 306, and the insertion tube 303 are thus disposed in tandem.

The transducer assembly 308 is positioned in the esophagus 301 to foster high-quality echocardiography images of a heart 309 of the patient. During an imaging and/or treatment procedure, the proximal end 304 of the insertion tube 303 is manipulated by a clinician's hands to guide the transducer assembly 308 in a desired position.

As noted above, the braid angle of the first section 310 is less than the braid angle of the middle section 311, and even less than the braid angle of the second section 312. This provides the first section 310 with a comparatively high durometer value; the middle section 311 with a lower durometer value than the first section 310; and the second section 312 with a durometer value that is lower than the middle section 311 or the first section 310. It is noted that the use of three sections having different durometer values is merely illustrative. In certain representative embodiments, the insertion tube 303 has two sections, with the transition from one section to another being continuous or continual or abrupt. In yet other embodiments, there are more than three sections, and again, the transition from one section to another being continuous or continual or abrupt. Finally, as noted above, instead of, or in addition to, the modification of the braid angle to tailor the desired changes in the durometer value, the desired changes in durometer value along the length of the insertion tube may be effected by selection of the tensile strength of the braid.

In a representative embodiment, the insertion tube 303 has a length of approximately 1 meter. The first section 310 is comparatively straight, and has a length between approximately 50 cm and approximately 40 cm. The middle section 311, which is an intermediate transition region, has a length between approximately 10 cm and approximately 20 cm. The second section 312 has a length between approximately 30-40 centimeters. Notably, the angular region 330 of section 312 is typically approximately 10 cm to approximately 20 cm in length, and transitions over an angle of approximately 80° and 100°.

The durometer value along the length of the insertion tube 303 is selected to transfer sufficient torque along the insertion tube 303, which has a reduced diameter/cross-section compared to known insertion tubes. The large durometer value of the insertion tube 303 compared to those of the bending neck 306 and transducer assembly 308 enables sufficient transfer of torque from the clinician at the proximal end 304 of the insertion tube 303 to the bending neck 306 without sacrificing flexibility in the bending neck 306 and the transducer assembly 308 to move within the esophagus. As such, by the present teachings, a more patient-friendly apparatus 300 is provided, which allows proper articulation of the transducer assembly 308 in order to provide quality images, and to carry out procedures with desired precision.

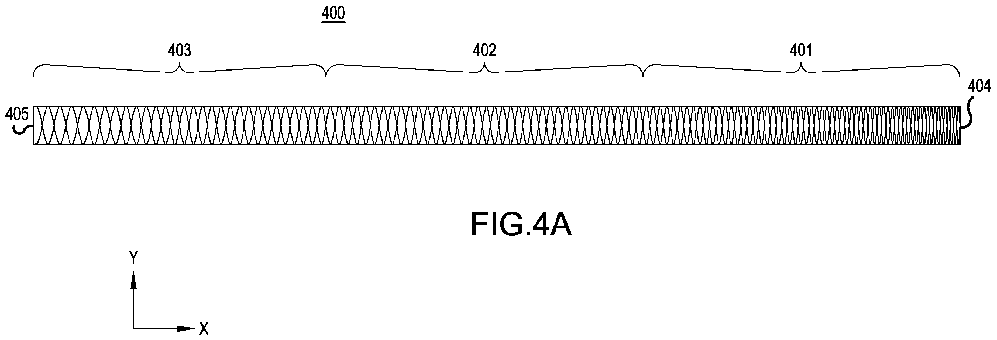

Fig. 4A is a side view of a portion of a braid 400 useful in the apparatuses described above, in accordance with a representative embodiment. Many aspects of the apparatuses are common to the apparatuses described above, and may not be repeated in the interest of clarity of description.

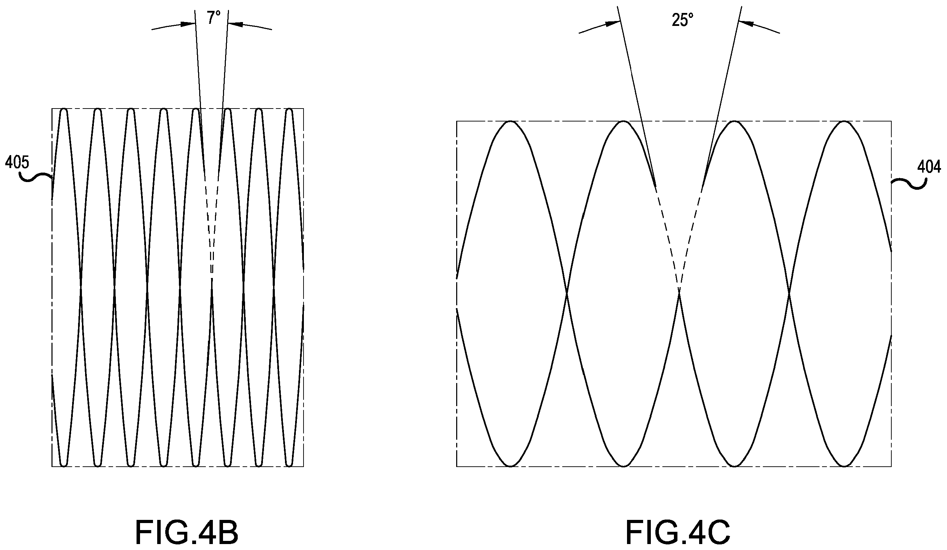

The braid 400 has a continuous change in braid angle along a length (x-direction in the coordinate system of Fig. 4A) from a first section 401, to a second 402, and a third section 403 between a first end 404 and a second end 405. In the representative embodiment, the braid angle increases in magnitude from the first end 404 to the second end 405, and gradually between the first~third sections 401-403. Just by way of illustration, Fig. 4B shows a section of the braid 400 near the first end 404 in the first section 401, and the braid angle at this section is approximately 7°. Fig. 4C shows another section of the braid 400 near the second end 405 in the third section 403, and the braid angle at this section is approximately 25°.

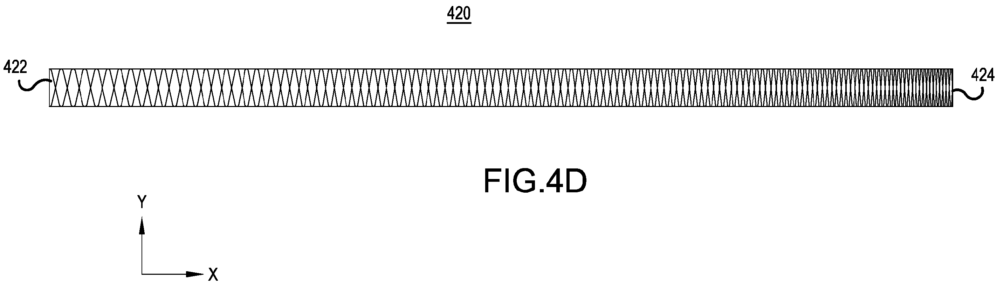

Fig. 4D is a side view of a portion of a braid 420 useful in the apparatuses described above, in accordance with a representative embodiment. Many aspects of the apparatuses are common to the apparatuses described above, and may not be repeated in the interest of clarity of description.

The braid 400 has a continuous change in braid angle along a length (x-direction in the coordinate system of Fig. 4D) between a first end 422 and a second end 424. As depicted in Fig. 4D, the braid angle decreases between the first end 422 and the second end 424.

Although apparatuses of the present teachings have been described with reference to several representative embodiments, it is understood that the words that have been used are words of description and illustration, rather than words of limitation. Changes may be made within the purview of the appended claims without departing from the scope of the patent. Although the illustrative apparatuses and systems that include these apparatuses of representative embodiments have been described with reference to particular means, materials and embodiments, the scope is not intended to be limited to the particulars disclosed, but extends to all functionally equivalent structures that are within the scope of the appended claims.

The illustrations of the embodiments described herein are intended to provide a general understanding of the structure of the various embodiments. The illustrations are not intended to serve as a complete description of all of the elements and features of the disclosure described herein. Many other embodiments may be apparent to those of skill in the art upon reviewing the disclosure. Other embodiments may be utilized and derived from the disclosure, such that structural and logical substitutions and changes may be made without departing from the scope of the disclosure. Additionally, the illustrations are merely representational and may not be drawn to scale. Certain proportions within the illustrations may be exaggerated, while other proportions may be minimized. Accordingly, the disclosure and the figures are to be regarded as illustrative rather than restrictive.

One or more embodiments of the disclosure may be referred to herein, individually and/or collectively, by the term "invention" merely for convenience and without intending to voluntarily limit the scope of this application to any particular invention or inventive concept. Moreover, although specific embodiments have been illustrated and described herein, it should be appreciated that any subsequent arrangement designed to achieve the same or similar purpose may be substituted for the specific embodiments shown. This disclosure is intended to cover any and all subsequent adaptations or variations of various embodiments. Combinations of the above embodiments, and other embodiments not specifically described herein, will be apparent to those of skill in the art upon reviewing the description.

In the foregoing Detailed Description, various features may be grouped together or described in a single embodiment for the purpose of streamlining the disclosure. This disclosure is not to be interpreted as reflecting an intention that the claimed embodiments require more features than are expressly recited in each claim. Rather, as the following claims reflect, inventive subject matter may be directed to less than all of the features of any of the disclosed embodiments. Thus, the following claims are incorporated into the Detailed Description, with each claim standing on its own as defining separately claimed subject matter.

In view of the foregoing, the present disclosure, through one or more of its various aspects, representative embodiments, and/or specific features or sub-components, is thus intended to bring out one or more of the advantages as specifically noted below. For purposes of explanation and not limitation, representative embodiments disclosing specific details are set forth in order to provide a thorough understanding of an embodiment according to the present teachings. However, other embodiments consistent with the present disclosure that depart from specific details disclosed herein remain within the scope of the appended claims.

## Claims

1. An apparatus (100,300), comprising:
a transducer assembly (108, 308);
a bending neck (106, 206, 306) connected to a proximal end (112) of the transducer assembly (108, 308); and
an insertion tube (103, 203, 303) disposed in tandem with the bending neck (106, 206, 306) and comprising a distal end (105, 205, 305) connected to a proximal end (111, 211, 321) of the bending neck (106,206, 306), and a proximal end (104, 204, 304) for manipulation of the transducer assembly (108, 308), the insertion tube (103, 203, 303) having a first durometer value at a first section (310), and a second durometer value at a second section (312), wherein the first durometer value is greater than the second durometer value, wherein the first section extends along a length of the insertion tube starting from the proximal end of the insertion tube, wherein the second section extends along a length of the insertion tube starting from the distal end of the insertion tube, wherein the first and second sections do not overlap, and wherein the insertion tube further comprises an interior channel (220);
**characterized in that** the insertion tube comprises an inner jacket (221) disposed circumferentially around the interior channel 220, wherein the inner jacket is a monocoil with helical shape, wherein a stiffness of the inner jacket is varied along the length of the insertion tube by selection of:
a gauge, a thickness or a pitch of the monocoil; or
by stacking and/or foregoing the monocoil at selected portions along the length of the monocoil that comprises the inner jacket.

2. The apparatus (100, 300) of claim 1, the insertion tube (103, 203, 303) further comprising a middle section (311) disposed between the first section (310) and the second section (312) of the insertion tube (103, 203, 303), wherein the insertion tube (103, 203, 303) has a third durometer value in the middle section (311), and the third durometer value is less than the first durometer value, and greater than the second durometer value.

3. The apparatus (100, 300) of claim 2, wherein the insertion tube (103, 203, 303) comprises a first braid (323) at its first section (310) the first braid (323) having a first durometer value, and a second braid (324) at its second section (312), the second braid (324) having a second durometer value, wherein the first durometer value is greater than the second durometer value.

4. The apparatus (100, 300) of claim 3, further comprising a third braid (325) disposed in the middle section (311) and having a third durometer value, wherein the third durometer value is less than the first durometer value, and greater than the second durometer value.

5. The apparatus (100, 300) of claim 3, wherein the insertion tube (103, 203, 303) further comprises an outer jacket (223) disposed around the first and second braids (323, 324).

6. The apparatus (100, 300) of claim 3, wherein the monocoil is a first monocoil, and the inner jacket (221) comprises a second monocoil in a region of the first braid (323).

7. The apparatus (100, 300) of claim 4, wherein the insertion tube (103, 203, 303) further comprises an outer jacket disposed around the first, second and third braids.

8. The apparatus (100, 300) of claim 3, wherein the first braid (323) has a first braid angle and a second braid (324) angle that is greater than the first braid angle.

9. The apparatus (100, 300) of claim 3, wherein the first braid (323) comprises a first plurality of wires, the second braid (324) comprises a second plurality of wires, and the first plurality of wires has a greater durometer value than the second plurality of wires.

10. An apparatus (100, 300), comprising:
a transducer assembly (108, 308);
a bending neck (106, 206, 306) connected to a proximal end (112) of the transducer assembly (108,308); and
an insertion tube (103, 203, 303) disposed in tandem with the bending neck (106, 206, 306) and having a distal end (105, 205, 305) connected to a proximal end (111, 211, 321) of the bending neck (106, 206, 306), and a proximal end (104, 204, 304) for manipulation of the transducer assembly (108, 308), the insertion tube (103, 203, 303) having a durometer value that decreases along its length between the proximal end (104, 204, 304) of the insertion tube (103, 203, 303) and distal end (105, 205, 305) of the insertion tube (103, 203, 303), wherein the insertion tube further comprises an interior channel (220);
**characterized in that** the insertion tube comprises an inner jacket (221) disposed circumferentially around the interior channel 220, wherein the inner jacket is a monocoil with helical shape, wherein a stiffness of the inner jacket is varied along the length of the insertion tube by selection of:
a gauge, a thickness or a pitch of the monocoil; or
by stacking and/or foregoing the monocoil at selected portions along the length of the monocoil that comprises the inner jacket.

11. The apparatus (100, 300) of claim 10, wherein the insertion tube (103, 203, 303) further comprises an outer jacket having a durometer value that decreases along its length between the proximal end (104, 204, 304) of the insertion tube (103, 203, 303) and distal end (105, 205, 305) of the insertion tube (103, 203, 303).

12. The apparatus (100, 300) of claim 11, wherein the insertion tube (103, 203, 303) comprises a braid (222) having a decreasing durometer value along its length between the proximal end (104, 204, 304) of the insertion tube (103, 203, 303) and the distal end (105, 205, 305) of the insertion tube (103, 203, 303).

13. The apparatus (100, 300) of claim 12, wherein a braid angle of the braid (322) decreases along its length between the proximal end (104, 204, 304) of the insertion tube (103, 203, 303) and the distal end (105, 205, 305) of the insertion tube (103, 203, 303).

14. The apparatus (100, 300) of claim 12, wherein the braid (222) comprises a plurality of wires, and a durometer value of the plurality of wires decreases between the proximal end (104, 204, 304) and the distal end (105, 205, 305) of the insertion tube (103, 203, 303).

## Patentansprüche

1. Einrichtung (100, 300), umfassend:
eine Wandleranordnung (108, 308);
einen Biegehals (106, 206, 306), der mit einem proximalen Ende (112) der Wandleranordnung (108, 308) verbunden ist; und
ein Einführrohr (103, 203, 303), das in Reihe mit dem Biegehals (106, 206, 306) angeordnet ist und ein distales Ende (105, 205, 305), das mit einem proximalen Ende (111, 211, 321) des Biegehalses (106, 206, 306) verbunden ist, und ein proximales Ende (104, 204, 304) zur Handhabung der Wandleranordnung (108, 308) umfasst, wobei das Einführrohr (103, 203, 303) an einem ersten Abschnitt (310) einen ersten Durometerwert und an einem zweiten Abschnitt (312) einen zweiten Durometerwert aufweist, wobei der erste Durometerwert größer ist als der zweite Durometerwert, wobei sich der erste Abschnitt entlang einer Länge des Einführrohrs erstreckt, beginnend am proximalen Ende des Einführrohrs, wobei sich der zweite Abschnitt entlang einer Länge des Einführrohrs erstreckt, beginnend am distalen Ende des Einführrohrs, wobei sich der erste und der zweite Abschnitt nicht überlappen, und wobei das Einführrohr weiter einen Innenkanal (220) umfasst;
**dadurch gekennzeichnet, dass** das Einführrohr einen inneren Mantel (221) umfasst, der umlaufend um den Innenkanal (220) angeordnet ist, wobei der innere Mantel eine Monospule mit Schraubenform ist, wobei eine Steifigkeit des inneren Mantels entlang der Länge des Einführrohrs durch eine Auswahl von Folgendem variiert wird:
eine Stärke, eine Dicke oder eine Steigung der Monospule; oder
durch Zusammenziehen und/oder Umgehen der Monospule an ausgewählten Abschnitten entlang der Länge der Monospule, die den inneren Mantel bildet.

2. Einrichtung (100, 300) nach Anspruch 1, wobei das Einführrohr (103, 203, 303) weiter einen Mittelabschnitt (311) umfasst, der zwischen dem ersten Abschnitt (310) und dem zweiten Abschnitt (312) des Einführrohrs (103, 203, 303) angeordnet ist, wobei das Einführrohr (103, 203, 303) in dem Mittelabschnitt (311) einen dritten Durometerwert aufweist und der dritte Durometerwert geringer als der erste Durometerwert und größer als der zweite Durometerwert ist.

3. Einrichtung (100, 300) nach Anspruch 2, wobei das Einführrohr (103, 203, 303) an seinem ersten Abschnitt (310) ein erstes Geflecht (323), wobei das erste Geflecht (323) einen ersten Durometerwert aufweist, und an seinem zweiten Abschnitt (312) ein zweites Geflecht (324) umfasst, wobei das zweite Geflecht (324) einen zweiten Durometerwert aufweist, wobei der erste Durometerwert größer als der zweite Durometerwert ist.

4. Einrichtung (100, 300) nach Anspruch 3, die weiter ein drittes Geflecht (325) umfasst, das in dem Mittelabschnitt (311) angeordnet ist und einen dritten Durometerwert aufweist, wobei der dritte Durometerwert geringer als der erste Durometerwert und größer als der zweite Durometerwert ist.

5. Einrichtung (100, 300) nach Anspruch 3,
wobei das Einführrohr (103, 203, 303) weiter einen äußeren Mantel (223) umfasst, der um das erste und das zweite Geflecht (323, 324) angeordnet ist.

6. Einrichtung (100, 300) nach Anspruch 3,
wobei die Monospule eine erste Monospule ist und der innere Mantel (221) eine zweite Monospule in einem Bereich des ersten Geflechts (323) umfasst.

7. Einrichtung (100, 300) nach Anspruch 4, wobei das Einführrohr (103, 203, 303) weiter einen äußeren Mantel umfasst, der um das erste, zweite und dritte Geflecht angeordnet ist.

8. Einrichtung (100, 300) nach Anspruch 3, wobei das erste Geflecht (323) einen ersten Flechtwinkel und einen zweiten Flechtwinkel (324) aufweist, der größer als der erste Flechtwinkel ist.

9. Einrichtung (100, 300) nach Anspruch 3, wobei das erste Geflecht (323) eine erste Vielzahl von Drähten umfasst, das zweite Geflecht (324) eine zweite Vielzahl von Drähten umfasst und die erste Vielzahl von Drähten einen größeren Durometerwert aufweist als die zweite Vielzahl von Drähten.

10. Einrichtung (100, 300), umfassend:
eine Wandleranordnung (108, 308);
einen Biegehals (106, 206, 306), der mit einem proximalen Ende (112) der Wandleranordnung (108, 308) verbunden ist; und
ein Einführrohr (103, 203, 303), das in Reihe mit dem Biegehals (106, 206, 306) angeordnet ist und ein distales Ende (105, 205, 305), das mit einem proximalen Ende (111, 211, 321) des Biegehalses (106, 206, 306) verbunden ist, und ein proximales Ende (104, 204, 304) zur Handhabung der Wandleranordnung (108, 308) aufweist, wobei das Einführrohr (103, 203, 303) einen Durometerwert aufweist, der entlang seiner Länge zwischen dem proximalen Ende (104, 204, 304) des Einführrohrs (103, 203, 303) und dem distalen Ende (105, 205, 305) des Einführrohrs (103, 203, 303) abnimmt, wobei das Einführrohr weiter einen Innenkanal (220) umfasst;
**dadurch gekennzeichnet, dass** das Einführrohr einen inneren Mantel (221) umfasst, der umlaufend um den Innenkanal (220) angeordnet ist, wobei der innere Mantel eine Monospule mit Schraubenform ist, wobei eine Steifigkeit des inneren Mantels entlang der Länge des Einführrohrs durch eine Auswahl von Folgendem variiert wird:
eine Stärke, eine Dicke oder eine Steigung der Monospule; oder
durch Zusammenziehen und/oder Umgehen der Monospule an ausgewählten Abschnitten entlang der Länge der Monospule, die den inneren Mantel bildet.

11. Einrichtung (100, 300) nach Anspruch 10, wobei das Einführrohr (103, 203, 303) weiter einen äußeren Mantel umfasst, der einen Durometerwert aufweist, der entlang seiner Länge zwischen dem proximalen Ende (104, 204, 304) des Einführrohrs (103, 203, 303) und dem distalen Ende (105, 205, 305) des Einführrohrs (103, 203, 303) abnimmt.

12. Einrichtung (100, 300) nach Anspruch 11, wobei das Einführrohr (103, 203, 303) ein Geflecht (222) umfasst, das einen abnehmenden Durometerwert entlang seiner Länge zwischen dem proximalen Ende (104, 204, 304) des Einführrohrs (103, 203, 303) und dem distalen Ende (105, 205, 305) des Einführrohrs (103, 203, 303) aufweist.

13. Einrichtung (100, 300) nach Anspruch 12, wobei ein Flechtwinkel des Geflechts (322) entlang seiner Länge zwischen dem proximalen Ende (104, 204, 304) des Einführrohrs (103, 203, 303) und dem distalen Ende (105, 205, 305) des Einführrohrs (103, 203, 303) abnimmt.

14. Einrichtung (100, 300) nach Anspruch 12, wobei das Geflecht (222) eine Vielzahl von Drähten umfasst und ein Durometerwert der Vielzahl von Drähten zwischen dem proximalen Ende (104, 204, 304) und dem distalen Ende (105, 205, 305) des Einführrohrs (103, 203, 303) abnimmt.

## Revendications

1. Appareil (100, 300) comprenant :
un ensemble transducteur (108, 308) ;
un segment souple (106, 206, 306) relié à une extrémité proximale (112) de l'ensemble transducteur (108, 308) ; et
un tube d'insertion (103, 203, 303) disposé en tandem avec le segment souple (106, 206, 306) et comprenant une extrémité distale (105, 205, 305) reliée à une extrémité proximale (111, 211, 321) du segment souple (106, 206, 306), et une extrémité proximale (104, 204, 304) pour la manipulation de l'ensemble transducteur (108, 308), le tube d'insertion (103, 203, 303) présentant une première valeur de duromètre au niveau d'une première section (310), et une deuxième valeur de duromètre au niveau d'une seconde section (312), dans lequel la première valeur de duromètre est supérieure à la deuxième valeur de duromètre, dans lequel la première section s'étend sur une longueur du tube d'insertion à partir de l'extrémité proximale du tube d'insertion, dans lequel la seconde section s'étend sur une longueur du tube d'insertion à partir de l'extrémité distale du tube d'insertion, dans lequel les première et seconde sections ne se chevauchent pas, et dans lequel le tube d'insertion comprend en outre un canal intérieur (220) ;
**caractérisé en ce que** le tube d'insertion comprend une gaine intérieure (221) disposée de manière circonférentielle autour du canal intérieur (220), dans lequel la gaine intérieure est un enroulement simple de forme hélicoïdale, dans lequel une rigidité de la gaine intérieure varie sur la longueur du tube d'insertion par sélection de :
un calibre, une épaisseur ou un pas de l'enroulement simple ; ou
par empilement de et/ou renoncement à l'enroulement simple au niveau de parties sélectionnées sur la longueur de l'enroulement simple qui comprend la gaine intérieure.

2. Appareil (100, 300) selon la revendication 1, le tube d'insertion (103, 203, 303) comprenant en outre une section médiane (311) disposée entre la première section (310) et la seconde section (312) du tube d'insertion (103, 203, 303), dans lequel le tube d'insertion (103, 203, 303) présente une troisième valeur de duromètre dans la section médiane (311), et la troisième valeur de duromètre est inférieure à la première valeur de duromètre, et supérieure à la deuxième valeur de duromètre.

3. Appareil (100, 300) selon la revendication 2, dans lequel le tube d'insertion (103, 203, 303) comprend une première tresse (323) au niveau de sa première section (310), la première tresse (323) présentant une première valeur de duromètre, et une deuxième tresse (324) au niveau de sa seconde section (312), la deuxième tresse (324) présentant une deuxième valeur de duromètre, dans lequel la première valeur de duromètre est supérieure à la deuxième valeur de duromètre.

4. Appareil (100, 300) selon la revendication 3, comprenant en outre une troisième tresse (325) disposée dans la section médiane (311) et présentant une troisième valeur de duromètre, dans lequel la troisième valeur de duromètre est inférieure à la première valeur de duromètre et supérieure à la deuxième valeur de duromètre.

5. Appareil (100, 300) selon la revendication 3,
dans lequel le tube d'insertion (103, 203, 303) comprend en outre une gaine extérieure (223) disposée autour des première et deuxième tresses (323, 324).

6. Appareil (100, 300) selon la revendication 3,
dans lequel l'enroulement simple est un premier enroulement simple, et la gaine intérieure (221) comprend un second enroulement simple dans une région de la première tresse (323).

7. Appareil (100, 300) selon la revendication 4, dans lequel le tube d'insertion (103, 203, 303) comprend en outre une gaine extérieure disposée autour des première, deuxième et troisième tresses.

8. Appareil (100, 300) selon la revendication 3, dans lequel la première tresse (323) présente un premier angle de tresse et un second angle de tresse (324) qui est supérieur au premier angle de tresse.

9. Appareil (100, 300) selon la revendication 3, dans lequel la première tresse (323) comprend une première pluralité de fils, la deuxième tresse (324) comprend une seconde pluralité de fils, et la première pluralité de fils présentent une valeur de duromètre supérieure à celle de la seconde pluralité de fils.

10. Appareil (100, 300) comprenant :
un ensemble transducteur (108, 308) ;
un segment souple (106, 206, 306) relié à une extrémité proximale (112) de l'ensemble transducteur (108, 308) ; et
un tube d'insertion (103, 203, 303) disposé en tandem avec le segment souple (106, 206, 306) et présentant une extrémité distale (105, 205, 305) reliée à une extrémité proximale (111, 211, 321) du segment souple (106, 206, 306), et une extrémité proximale (104, 204, 304) pour la manipulation de l'ensemble transducteur (108, 308), le tube d'insertion (103, 203, 303) présentant une valeur de duromètre qui diminue sur sa longueur entre l'extrémité proximale (104, 204, 304) du tube d'insertion (103, 203, 303) et l'extrémité distale (105, 205, 305) du tube d'insertion (103, 203, 303), dans lequel le tube d'insertion comprend en outre un canal intérieur (220) ;
**caractérisé en ce que** le tube d'insertion comprend une gaine intérieure (221) disposée de manière circonférentielle autour du canal intérieur (220), dans lequel la gaine intérieure est un enroulement simple de forme hélicoïdale, dans lequel une rigidité de la gaine intérieure varie sur la longueur du tube d'insertion par sélection de :
un calibre, une épaisseur ou un pas de l'enroulement simple ; ou
par empilement de et/ou renoncement à l'enroulement simple au niveau de parties sélectionnées sur la longueur de l'enroulement simple qui comprend la gaine intérieure.

11. Appareil (100, 300) selon la revendication 10, dans lequel le tube d'insertion (103, 203, 303) comprend en outre une gaine extérieure présentant une valeur de duromètre qui diminue sur sa longueur entre l'extrémité proximale (104, 204, 304) du tube d'insertion (103, 203, 303) et l'extrémité distale (105, 205, 305) du tube d'insertion (103, 203, 303).

12. Appareil (100, 300) selon la revendication 11, dans lequel le tube d'insertion (103, 203, 303) comprend une tresse (222) présentant une valeur de duromètre décroissante sur sa longueur entre l'extrémité proximale (104, 204, 304) du tube d'insertion (103, 203, 303) et l'extrémité distale (105, 205, 305) du tube d'insertion (103, 203, 303).

13. Appareil (100, 300) selon la revendication 12, dans lequel un angle de tresse de la tresse (322) diminue sur sa longueur entre l'extrémité proximale (104, 204, 304) du tube d'insertion (103, 203, 303) et l'extrémité distale (105, 205, 305) du tube d'insertion (103, 203, 303).

14. Appareil (100, 300) selon la revendication 12, dans lequel la tresse (222) comprend une pluralité de fils, et une valeur de duromètre de la pluralité de fils diminue entre l'extrémité proximale (104, 204, 304) et l'extrémité distale (105, 205, 305) du tube d'insertion (103, 203, 303).
